(19) Europäisches Patentamt
European Patent Office
Office européen des brevets

(11) Veröffentlichungsnummer : **0 055 215**
**B1**

(12) # EUROPÄISCHE PATENTSCHRIFT

(45) Veröffentlichungstag der Patentschrift : **30.01.85**

(51) Int. Cl.[4] : **C 07 D207/26**, C 07 D207/273, A 01 N 43/36

(21) Anmeldenummer : **81810496.0**

(22) Anmeldetag : **14.12.81**

(54) **Neue Fluorpyrrolidinone, Verfahren zu deren Herstellung, sie enthaltende herbizide Mittel und deren Verwendung.**

(30) Priorität : **19.12.80 CH 9401/80**

(43) Veröffentlichungstag der Anmeldung : **30.06.82 Patentblatt 82/26**

(45) Bekanntmachung des Hinweises auf die Patenterteilung : **30.01.85 Patentblatt 85/05**

(84) Benannte Vertragsstaaten : **BE CH DE FR GB IT LI NL**

(56) Entgegenhaltungen :
**DE-A- 2 612 731**
**US-A- 3 488 732**
**US-A- 4 132 713**

(73) Patentinhaber : **CIBA-GEIGY AG**
**Postfach**
**CH-4002 Basel (CH)**

(72) Erfinder : **Rempfler, Hermann, Dr.**
**Brücklismattstrasse 16**
**CH-4107 Ettingen (CH)**
Erfinder : **Meyer, Willy**
**Talweg 49**
**CH-4125 Riehen (CH)**

**EP 0 055 215 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Fluorpyrrolidinone mit herbizider Wirkung, Verfahren zu deren Herstellung, sie als Wirkstoff enthaltende Mittel sowie deren Verwendung zur selektiven Unkrautbekämpfung in verschiedenen Kulturen wie z. B. Getreide, Reis, Mais, Soja und Baumwolle.

Die neuen Fluorpyrrolidinone entsprechen der allgemeinen Formel I

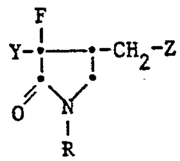

(I)

worin

R $C_1$-$C_4$-Alkyl ; $C_3$-$C_4$-Alkenyl ; Alkoxyalkyl mit insgesamt höchstens 5 Kohlenstoffatomen ; gegebenenfalls durch Halogen, $C_1$-$C_4$-Alkoxy oder $C_1$-$C_4$-Alkyl substituiertes Benzyl ; oder ein- oder mehrfach durch Halogen, Nitro, Cyan, Methyl, Halogenmethyl, $C_1$-$C_4$-Alkoxy, $C_1$-$C_4$-Halogenalkoxy, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl substituiertes Phenyl bedeutet und

Y für Wasserstoff, $C_1$-$C_4$-Alkyl, Fluor, Chlor oder Brom und

Z für Chlor oder Brom stehen.

Unter Alkyl oder Alkylteilen von Substituenten wie Alkoxy, Alkoxyalkyl, Alkylthio, Alkylsulfinyl, Alkylsulfonyl oder den entsprechenden Halogenalkylsubstituenten sind zu verstehen : Methyl, Aethyl, n-Propyl, i-Propyl sowie die vier isomeren Butylreste. Halogen als Substituent oder Teil eines Substituenten steht für Fluor, Chlor, Brom und Jod, vorzugsweise aber für Fluor und Chlor.

Entsprechend sind beispielsweise Alkoxyreste : Methoxy, Aethoxy und Propyloxy ; Halogenalkylreste : Fluormethyl, Chlormethyl, Trifluormethyl, Perfluoräthyl oder Difluormethyl, vorzugsweise Trifluormethyl ; Alkoxyalkylreste : Methoxymethyl, Aethoxymethyl, Methoxyäthyl oder Aethoxyäthyl ; Halogenalkoxyreste : Trifluormethoxy, Difluormethoxy, Perfluoräthoxy ; Alkylthio- und Halogenalkylthioreste : Methylthio, Aethylthio, Trifluormethylthio, Difluormethylthio, Chlormethylthio, Perfluoräthylthio oder Chlorfluormethylthio ; Alkylsulfinyl- und Halogenalkylsulfinylreste : Methylsulfinyl, Aethylsulfinyl, Trifluormethylsulfinyl, Difluormethylsulfinyl oder Perfluoräthylsulfinyl ; und Alkylsulfonyl- und Halogenalkylsulfonylreste : Methylsulfonyl, Aethylsulfonyl, Trifluorsulfonyl, Difluormethylsulfonyl oder Perfluoräthylsulfonyl.

Beispiele für Alkenyl sind : Allyl, Methallyl, 2-Butenyl oder 3-Butenyl. Herbizid wirksame Chlor- und Brompyrrolidinone ähnlicher Struktur sind aus der deutschen Offenlegungsschrift 2 612 731 bekannt geworden. Ein verallgemeinertes Herstellungsverfahren dieser Verbindungen ist im US-Patent 4 132 713 beschrieben. Gegenüber diesen Verbindungen besitzen die erfindungsgemässen Fluorpyrrolidinone eine bessere Selektivität in Nutzpflanzenkulturen wie Getreide, Reis, Mais, Soja und Baumwolle bei post- und preemergenter Anwendung, insbesondere aber bei der Anwendung im Vorauflaufverfahren.

Wegen ihrer guten Wirksamkeit sind die Verbindungen hervorzuheben, in denen R einen ein- oder mehrfach durch Halogen, Nitro, Cyan, Methoxy, Halogenmethoxy, Methyl, Halogenmethyl, Halogenmethylthio, Halogenmethylsulfinyl oder Halogenmethylsulfonyl substituierten Phenylkern, Y Wasserstoff, Methyl, Chlor oder Brom und Z Chlor oder Brom bedeuten.

Eine weitere Bevorzugung ist bei den Verbindungen der Formel I darin zu sehen, dass R einen ein- oder mehrfach durch Halogen, Halogenmethyl oder Halogenmethoxy substituierten Phenylkern, Y Wasserstoff oder Chlor bedeuten und Z für Chlor steht.

Ganz besonders bevorzugt sind die Verbindungen der Formel I, in denen R einen in 3-Stellung durch Halogen, Trifluormethyl, Trifluormethoxy oder Difluormethoxy substituierten Phenylkern, Y Wasserstoff oder Chlor und Z Chlor bedeuten.

Als bevorzugte Einzelverbindung ist zu nennen : N-(3-Trifluormethylphenyl)-3-fluor-4-chlormethyl-pyrrolidin-2-on unter Einschluss ihrer diastereomeren Formen.

Die Verbindungen der Formel I werden erfindungsgemäss analog zum in der DOS 2 612 731 beschriebenen Verfahren hergestellt, indem man ein N-Allyl-acetamid der Formel II,

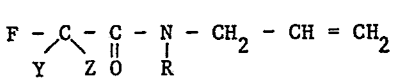

(II)

worin R, Y und Z die unter Formel I angegebene Bedeutung haben, in Gegenwart eines Katalysators in einem inerten Lösungsmittel erhitzt.

Die Reaktionstemperaturen liegen vorteilhafterweise zwischen 50° und 180 °C, insbesondere aber zwischen 120° und 170 °C.

Als Katalysatoren kommen Uebergangsmetallionen, wie z. B. Ionen von Vanadium, Eisen, Kupfer, Molybdän, Ruthenium oder Silber, oder deren Komplexe, wie z. B. Kupfer-I-chlorid-Piperidin, Kupfer-I-chlorid-2,2'-Dipyridyl oder Rutheniumchlorid-Triphenylphosphin, in Betracht.

Bevorzugte inerte Lösungsmittel sind z. B. Diäthylenglykoldimethyläther, Dimethylformamid, Dimethylsulfoxid, Mesitylen, Dekalin oder Tetralin.

Die Ausgangsverbindungen der Formel II können in allgemein bekannter Weise aus bekanten Allylaminen der Formel III

$$R—NH—CH_2—CH = CH_2 \qquad (III)$$

durch Umsetzen mit Fluoressigsäure-Halogeniden der Formel IV

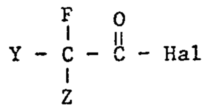

(IV)

in Gegenwart von Basen erhalten werden.

In den Formeln III und IV haben R, Y und Z die unter Formel I angegebene Bedeutung und Hal steht für Chlor oder Brom.

Als Basen können beispielsweise verwendet werden : Trialkylamine wie Triäthylamin ; Hydroxide wie Natrium- oder Kaliumhydroxid ; Carbonate wie Natrium- oder Kaliumcarbonat ; Hydrogencarbonate wie Natriumhydrogencarbonat oder Hydride wie Natrium- oder Calciumhydrid.

Bei der Ringschlussreaktion (I→II) können beide möglichen Konfigurationsisomere der erfinderischen Verbindungen der Formel I entstehen : das cis- und das trans-Isomere. Im allgemeinen entsteht das Isomerengemisch, das durch geeignete Aufarbeitungsmethoden, wie z. B. fraktionierte Kristallisation, Destillation, Chromatographie oder Extraktion, in seine Komponenten aufgetrennt werden kann. Soweit es im folgenden nicht besonders erwähnt ist, ist unter einer Verbindung stets das Isomerengemisch zu verstehen.

Die Verbindungen der Formel I haben ausgezeichnete herbizide Eigenschaften gegen monokotyle und dikotyle Unkräuter. Diese Wirkstoffe können sowohl im Vorauflauf- als auch im Nachauflaufverfahren angewendet werden. Besonders geeignet zum Bekämpfen von Unkräutern sind die Wirkstoffe der Formel I in Kulturpflanzungen wie Getreide, Reis, Mais, Soja und Baumwolle weil sie in hohem Masse selektiv wirken. Es werden dabei die Unkräuter vernichtet, die Nutzpflanzen aber geschont oder nur geringfügig in ihrer Entwicklung beeinflusst.

Als ganz besonders geeignet haben sich die Wirkstoffe der Formel I erwiesen, wann man sie zur selektiven Unkräutbekämpfung in den genannten Kulturen im Vorauflaufverfahren verwendet.

Die beiden konfigurationsisomeren Formen der Wirkstoffe haben gleiche oder auch verschiedene biologische Aktivität.

Die Verbindungen der Formel I werden in unveränderter Form oder vorzugsweise als Mittel mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen. (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtiogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsufonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylenpolyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl- oder niederige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgender Publikation beschrieben : « Mc Cutcheon's Detergents and Emulsifiers Annual » MC Publishing Corp., Ridgewood, New Jersey, 1979.

Sisley and Wood, « Encyclopedia of Surface Active Agents », Chemical Publishing Co., Inc. New York, 1964.

Die pestiziden Zubereiterungen enthalten in der Regel 0,1 bis 90 % Wirkstoff der Formel I, 10 bis 99 % eines festen oder flüssigen Zusatzstoffes und 1 bis 25 % eines Tensides.

Insbesondere setzen sich bevorzugte Formulierungen folgendermassen zusammen : (% = Gewichtsprozent).

Lösungen

| | |
|---|---|
| Aktiver Wirkstoff : | 5 bis 90 %, vorzugsweise 10 bis 80 % |
| Lösungsmittel : | 95 bis 5 %, vorzugsweise 90 bis 0 % |
| oberflächenaktives Mittel : | 1 bis 25 %, vorzugsweise 2 bis 20 %. |

Emulgierbare Konzentrate :

| | |
|---|---|
| Aktiver Wirkstoff : | 10 bis 50 %, bevorzugt 10 bis 40 % |
| oberflächenaktives Mittel : | 5 bis 30 %, vorzugsweise 10 bis 20 % |
| flüssiges Trägermittel : | 20 bis 95 %, vorzugsweise 40 bis 80 %. |

Stäube

| | |
|---|---|
| Aktiver Wirkstoff : | 0,5 bis 10 %, vorzugsweise 2 bis 8 % |
| festes Trägermittel : | 99,5 bis 90 %, vorzugsweise 98 bis 92 %. |

Suspensions-Konzentrate

| | |
|---|---|
| Aktiver Wirkstoff : | 5 bis 75 %, vorzugsweise 10 bis 50 % |
| Wasser : | 94 bis 25 %, vorzugsweise 90 bis 30 % |
| oberflächenaktives Mittel : | 1 bis 40 %, vorzugsweise 2 bis 30 %. |

Benetzbare Pulver

| | |
|---|---|
| Aktiver Wirkstoff : | 5 bis 90 %, vorzugsweise 10 bis 80 % und insbesondere 20 bis 60 % |
| oberflächenaktives Mittel : | 1 bis 20 %, vorzugsweise 1 bis 15 % |
| festes Trägermittel : | 5 bis 90 %, vorzugsweise 30 bis 70 %. |

Granulate

| | |
|---|---|
| Aktiver Wirkstoff : | 1 bis 30 %, vorzugsweise 3 bis 15 % |
| festes Trägermittel : | 99 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,1 bis 10 kg AS/ha, vorzugsweise 0,25 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgende Beispiele dienen zur näheren Erläuterung der Erfindung, ohne dieselbe einzuschränken. Die Temperaturen sind in Celsiusgraden angegeben. Prozente und Teile beziehen sich auf Gewicht. Der Druck ist in Millibar (mb) angegeben.

## Herstellungsbeispiele

### Beispiel 1

a) Herstellung eines Zwischenproduktes

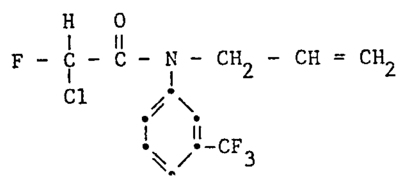

N-Allyl-N-chlorfluoracetyl-3-trifluormethyl-anilin

Zur auf 10° bis 15 °C gekühlten Lösung von 116,7 g N-Allyl-3-trifluormethyl-anilin und 60,0 g Triäthylamin in 700 ml Diäthyläther lässt man 86,0 g Chlorfluoracetylchlorid zutropfen. Nachdem das Reaktionsgemisch für weitere 3 Stunden bei Raumtemperatur gerührt worden ist, wird das ausgefallene Salz abfiltriert, das Filtrat zuerst mit 5 %iger Salzsäure, dann mit 5 %iger Natriumhydroxidlösung gewaschen, getrocknet und eingedampft. Die Hochvakuumdestillation des Rückstands ergibt 138,5 g N-Allyl-N-chlorfluoracetyl-3-trifluormethyl-anilin, Sdp. 93°-94 °C/0,02 mb.

b) Herstellung eines Endproduktes

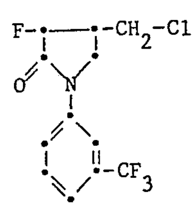

Verb. Nr. 35

N-(3-Trifluormethylphenyl)-3-fluor-4-chlormethylpyrrolidin-2-on

Zur siedenden Lösung von 1,0 g Kupfer-I-chlorid und 1,5 g 2,2'-Dipyridyl in 50 ml Diäthylenglykoldimethyläther lässt man innerhalb einer halben Stunde 60,0 g N-Allyl-N-chlorfluoracetyl-3-trifluormethylanilin zutropfen. Nachdem die Reaktionsmischung für weitere 5 Stunden am Rückfluss gekocht worden ist, wird sie nach dem Abkühlen mit einem Gemisch aus Diäthyläther und 5 %iger Salzsäure aufgenommen. Die Aetherphase wird mit Wasser gewaschen, getrocknet und eingedampft. Die Hochvakuumdestillation des Rückstandes ergibt 52 g N-(3-Trifluormethylphenyl)-3-fluor-4-chlormethyl-pyrrolidin-2-on, Sdp. 115°-120 °C/0,002 mb, welches nach einiger Zeit zu einem Kristallbrei erstarrt.

Beispiel 2

Trennung der Konfigurationsisomeren

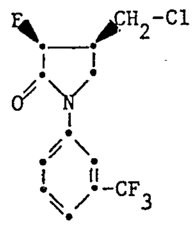

und

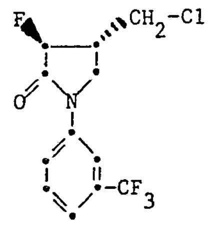

« cis », Verb. Nr. 50

cis-N-(3-Trifluormethylphenyl)-3-fluor-4-chlormethyl-pyrrolidin-2-on

« trans », Verb. Nr. 51

trans-N-(3-Trifluormethylphenyl)-3-fluor-4-chlormethyl-pyrrolidin-2-on

10 g des in Beispiel 1 erhaltenen cis-trans-Isomerengemisches ergeben bei säulenchromatographischer Trennung am Silicagel mit Essigsäureäthylester-Hexan-Gemisch (1 : 3) 2,5 g cis-N-(3-Trifluormethylphenyl)-3-fluor-4-chlormethyl-pyrrolidin-2-on, Smp. 54°-55 °C und 7,5 g trans-N-(3-Trifluormethylphenyl)-3-fluor-4-chlormethyl-pyrrolidin-2-on, Smp. 72°-74 °C.

Die nach den Herstellungsbeispielen erhaltenen und analog hergestellte Verbindungen sind in der anschliessenden Tabelle 1 aufgeführt :

Tabelle 1

Verbindungen der Formel I

| Nr. | R | Y | Z | phys. Daten |
|---|---|---|---|---|
| 1 | $CH_2=CH-CH_2-$ | Cl | Cl | |
| 2 | $n-C_4H_9-$ | H | Cl | |
| 3 | $C_6H_5-CH_2-$ | H | Cl | $n_D^{30}$ 1.4898 |
| 4 | $3-Cl-C_6H_4-CH_2-$ | H | Cl | $n_D^{30}$ 1.5391 |
| 5 | $3-Cl-C_6H_4-CH_2-$ | Cl | Cl | |
| 6 | $CH_3-O-CH_2-CH_2-$ | H | Cl | |
| 7 | $3-F-C_6H_4-$ | H | Cl | $n_D^{30}$ 1.5439 |
| 8 | $3-Cl-C_6H_4-$ | H | Cl | $n_D^{30}$ 1.5542 |
| 9 | $3-Br-C_6H_4$ | H | Cl | Smp. 79.93°C |
| 10 | $3-J-C_6H_4-$ | H | Cl | Smp. 88-102°C |
| 11 | $3-CH_3-C_6H_4-$ | H | Cl | $n_D^{25}$ 1.5348 |
| 12 | $3-OCH_3-C_6H_4-$ | H | Cl | Smp. 84-87°C |
| 13 | $3-CN-C_6H_4-$ | H | Cl | Smp. 124-130°C |
| 14 | $3-NO_2-C_6H_4-$ | H | Cl | Smp. 80-90°C |

Tabelle 1 (Fortsetzung)

| Nr. | R | Y | Z | phys. Daten |
|---|---|---|---|---|
| 15 | $4-F-C_6H_4-$ | H | Cl | Smp. 96-114°C |
| 16 | $3-CF_3-4-Cl-C_6H_3-$ | H | Cl | Smp. 107-109°C |
| 17 | $3-CF_3-6-Cl-C_6H_3-$ | H | Cl | |
| 18 | $3-CF_3-5-CF_3-C_6H_3-$ | H | Cl | Sdp. 108-110°C/0,02 mb |
| 19 | $3-NO_2-6-OCH_3-C_6H_3-$ | H | Cl | Smp. 122-128°C |
| 20 | $3-OCH_3-5-OCH_3-C_6H_3-$ | Cl | Cl | Smp. 87-94°C |
| 21 | $3-OCH_3-6-OCH_3-C_6H_3-$ | H | Cl | $n_D^{30}$= 1,5481 |
| 22 | $3-Cl-6-OCH_3-C_6H_3-$ | H | Cl | $n_D^{30}$= 1,5457 |
| 23 | $3-Cl-5-Cl-C_6H_3-$ | H | Cl | halbkristallin |
| 24 | $3-F-6-F-C_6H_3-$ | H | Cl | Smp. 117-124°C |
| 25 | $3-NO_2-4-CH_3-C_6H_3-$ | H | Cl | Smp. 98-104°C |
| 26 | $3-Cl-4-OCH_3-6-OCH_3-C_6H_2-$ | H | Cl | Smp. 159-164°C |
| 27 | $3-Cl-4-Cl-6-Cl-C_6H_2-$ | H | Cl | Smp. 105-113°C |
| 28 | $C_6F_5-$ | H | Cl | Smp. 89-94°C |
| 29 | $3-OCF_3-C_6H_4-$ | H | Cl | $n_D^{30}$= 1,4920 |
| 30 | $3-CHF_2-O-C_6H_4-$ | H | Cl | $n_D^{30}$= 1,5268 |
| 31 | $3-SCF_3-C_6H_4-$ | H | Cl | |
| 32 | $3-CHF_2-S-C_6H_4-$ | H | Cl | |
| 33 | $3-CHF_2-SO-C_6H_4-$ | H | Cl | |
| 34 | $3-CHF_2-SO_2-C_6H_4-$ | H | Cl | |
| 35 | $3-CF_3-C_6H_4-$ | H | Cl | Sdp. 115-120°C/0,002 mb |
| 36 | $3-CF_3-C_6H_4-$ | Cl | Cl | |
| 37 | $3-CHF_2-O-C_6H_4-$ | Cl | Cl | |
| 38 | $3-Cl-C_6H_4-$ | Cl | Cl | |
| 39 | $3-Br-C_6H_4-$ | Cl | Cl | |
| 40 | $3-CF_3-C_6H_4-$ | H | Br | |
| 41 | $3-Cl-C_6H_4-$ | H | Br | |
| 42 | $3-CH_3-C_6H_4-$ | H | Br | |
| 43 | $3-CF_3-C_6H_4-$ | Br | Br | |
| 44 | $3-Cl-C_6H_4-$ | Br | Br | |
| 45 | $3-Br-C_6H_4-$ | Br | Br | |

Tabelle 1 (Fortsetzung)

| Nr. | R | Y | Z | phys. Daten |
|---|---|---|---|---|
| 46 | $3-CF_3-C_6H_4-$ | $CH_3$ | Cl | |
| 47 | $3-CHF_2-O-C_6H_4-$ | $CH_3$ | Cl | |
| 48 | $3-Cl-C_6H_4-$ | $CH_3$ | Cl | |
| 49 | $3-Cl-5-Cl-C_6H_3-$ | $CH_3$ | Cl | |
| 50 | $3-CF_3-C_6H_4-$ | H | Cl | cis-Isomeres Smp. 54-55°C |
| 51 | $3-CF_3-C_6H_4-$ | H | Cl | trans-Isomeres Smp. 72-74°C |

## Formulierungsbeispiele

### Beispiel 1

Formulierungsbeispiele für flüssige Wirkstoffe der Formel I (% = Gewichtsprozent)

| a) Emulsions-Konzentrate | a) | b) | c) |
|---|---|---|---|
| Wirkstoff Nr. 30 | 20 % | 40 % | 50 % |
| Ca-Dodecylbenzolsulfonat | 5 % | 8 % | 5,8 % |
| Ricinusölpolyäthylenglykoläther (36 Mol AeO) | 5 % | — | — |
| Tributylphenolpolyäthylenglykoläther (30 Mol AeO) | — | 12 % | 4,2 % |
| Cyclohexanon | — | 15 % | 20 % |
| Xylolgemisch | 70 % | 25 % | 20 % |

Aus solchen Konzentraten können durch Verdünnen mit Wasser Emulsionen jeder gewünschte. Konzentrationen hergestellt werden.

| b) Lösungen | a) | b) | c) | d) |
|---|---|---|---|---|
| Wirkstoff Nr. 11 | 80 % | 10 % | 5 % | 95 % |
| Aethylenglykolmonomethyläther | 20 % | — | — | — |
| Polyäthylenglykol MG 400 | — | 70 % | — | — |
| N-Methyl-2-pyrrolidon | — | 20 % | — | — |
| Epoxydiertes Kokosnussöl | — | — | 1 % | 5 % |
| Benzin (Siedegrenzen 160-190 °C) | — | — | 94 % | — |

Die Lösungen sind zur Anwendung in Form kleinster Tropfen geeignet.

| c) Granulate | a) | b) |
|---|---|---|
| Wirkstoff Nr. 8 | 5 % | 10 % |
| Kaolin | 94 % | — |
| Hochdisperse Kieselsäure | 1 % | — |
| Attapulgit | — | 90 % |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff Nr. 8 | 2 % | 5 % |
| Hochdisperse Kieselsäure | 1 % | 5 % |
| Talkum | 97 % | — |
| Kaolin | — | 90 % |

Durch inniges Vermischen der Trägerstoffe mit dem Wirkstoff erhält man gebrauchsfertige Stäubemittel.

Beispiel 2

Formulierungsbeispiele für feste Wirkstoffe der Formel I (% = Gewichtsprozent)

a) Spritzpulver

| | a) | b) |
|---|---|---|
| Wirkstoff Nr. 35 | 20 % | 60 % |
| Na-Ligninsulfonat | 5 % | 5 % |
| Na-Laurylsulfat | 3 % | — |
| Na-Diisobutylnaphthalinsulfonat | — | 6 % |
| Octylphenolpolyäthylenglykoläther (7-8 Mol AeO) | — | 2 % |
| Hochdisperse Kieselsäure | 5 % | 27 % |
| Kaolin | 67 % | — |

Der Wirkstoff wird mit den Zusatzstoffen gut vermischt und in einer geeigneten Mühle gut vermahlen. Man erhält Spritzpulver, die sich mit Wasser zur Suspensionen jeder gewünschten Konzentration verdünnen lassen.

b) Emulsions-Konzentrat

| | |
|---|---|
| Wirkstoff Nr. 50 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff Nr. 51 | 5 % | 8 % |
| Talkum | 95 % | — |
| Kaolin | — | 92 % |

Man erhält anwendungsfertige Stäubemittel, indem der Wirkstoff mit dem Träger vermischt und auf einer geeigneten Mühle vermahlen wird.

d) Extruder Granulat

| | |
|---|---|
| Wirkstoff Nr. 19 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff Nr. 12 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff Nr. 10 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele :

Zum Nachweis der Brauchbarkeit als Herbizide (pre- und post-emergent) dienen folgende Testmethoden :

Beispiel 1

Nachweis der Herbizidwirkung vor dem Auflaufen der Pflanzen

Im Gewächshaus werden Pflanzensamen in Blumentöpfe von 12-15 cm Durchmesser gesät. Unmittelbar danach wird die Erdoberfläche mit einer wässrigen Dispersion oder Lösung der Wirkstoffe behandelt. Es werden Konzentrationen von 4 kg Wirkstoffmengen pro Hektar angewendet. Die Töpfe werden dann im Gewächshaus bei einer Temperatur von 22-25 °C und 50-70 % relativer Luftfeuchtigkeit gehalten. Nach 3 Wochen wird der Versuch ausgewertet und die Wirkung nach folgendem Massstabbeurteilt :

1 : Pflanzen nicht gekeimt oder total abgestorben,
2-3 : sehr starke Wirkung,
4-6 : mittlere Wirkung,
7-8 : geringe Wirkung,
9 : keine Wirkung (wie unbehandelte Kontrolle).

Pre-emergente Wirkung

Aufwandmenge : 4 kg Wirksubstanz/Hektar

| Verb.. Nr. | Avena | Setaria | Sinapis | Stellaria |
|---|---|---|---|---|
| 8 | 8 | 1 | 1 | 1 |
| 9 | 8 | 1 | 2 | 2 |
| 10 | 8 | 1 | 6 | 1 |
| 11 | 9 | 2 | 5 | 2 |
| 12 | 8 | 3 | 4 | 2 |
| 13 | 5 | 2 | 1 | 1 |
| 16 | 6 | 1 | 2 | 1 |
| 29 | 5 | 1 | 1 | 2 |
| 30 | 7 | 1 | 2 | 3 |
| 50 | 1 | 1 | 1 | 1 |
| 51 | 2 | 1 | 1 | 1 |

Beispiel 2

Nachweis der Selektivität bei Vorauflaufanwendung

In der gleiche Versuchsanordnung wie im Beispiel 1 werden eine grössere Anzahl von Pflanzensamen mit verschiedenen Aufwandmengen an Wirksubstanz behandelt. Die Auswertung erfolgte nach dem gleichen Massstab.

Die geprüften Verbindungen der Formel I zeigten in diesem Versuch ausgezeichnete Wirkung gegen zweikeimblättrige und auch gegen die meisten grasartigen Unkräuter, während Kulturpflanzen wie Getreide, Mais, Hirse, Reis, Soja und Baumwolle bei angemessener Aufwandmenge nicht oder nur unbedeutend geschädigt wurden.

(Siehe Tabelle Seite 11 ff.)

Pre-emergente Herbizid-Wirkung

| Wirkung | | | | | | | | | | | | |
|---|---|---|---|---|---|---|---|---|---|---|---|---|
| Aufwandmenge kg AS/ha | Verb. Nr. 35 | | | | Verb. Nr. 50 | | | | Verb. Nr. 51 | | | |
| Testpflanze | 4 | 2 | 1 | 0,5 | 4 | 2 | 1 | 0,5 | 4 | 2 | 1 | 0,5 |
| Gerste | 3 | 3 | 8 | 9 | 1 | 1 | 6 | 6 | 2 | 3 | 6 | 9 |
| Weizen | 3 | 4 | 9 | 9 | 1 | 2 | 3 | 8 | 1 | 7 | 8 | 9 |
| Mais | 3 | 5 | 8 | 9 | 1 | 2 | 6 | 8 | 1 | 2 | 2 | 8 |
| Sorghum hybr. | 1 | 2 | 7 | 9 | 1 | 1 | 9 | 9 | 1 | 1 | 4 | 7 |
| Reis trocken | 4 | 5 | 9 | 9 | 1 | 2 | 4 | 9 | 1 | 2 | 7 | 9 |
| Avena fatua | 1 | 2 | 7 | 8 | 1 | 1 | 2 | 3 | 1 | 1 | 2 | 7 |
| Bromus tectorum | 1 | 1 | 4 | 7 | 1 | 1 | 2 | 2 | 1 | 1 | 2 | 4 |
| Lolium perenne | 1 | 1 | 2 | 8 | 1 | 1 | 2 | 3 | 1 | 1 | 2 | 7 |
| Alopecurus myos. | 1 | 1 | 2 | 2 | 1 | 1 | 1 | 2 | 1 | 1 | 1 | 2 |
| Digitaria sang. | 1 | 1 | 1 | 1 | 1 | 1 | 1 | 1 | 1 | 1 | 1 | 1 |
| Echinochloa c.g. | 1 | 1 | 1 | 2 | 1 | 1 | 1 | 1 | 1 | 1 | 1 | 1 |
| Sorghum halep. | 1 | 1 | 1 | 1 | 1 | 1 | 1 | 1 | 1 | 1 | 3 | 5 |
| Rottboellia ex. | 1 | 8 | 8 | 9 | 1 | 3 | 9 | 9 | 1 | 1 | 6 | 9 |
| Cyperus escul. | 2 | 6 | 9 | 9 | 2 | 3 | 3 | 9 | 1 | 1 | 2 | 7 |
| Soja | 2 | 4 | 4 | 8 | 2 | 3 | 6 | 8 | 2 | 3 | 6 | 7 |
| Baumwolle | 8 | 8 | 9 | 9 | 6 | 6 | 9 | 9 | 8 | 9 | 9 | 9 |
| Zuckerrübe | 1 | 1 | 1 | 1 | 1 | 1 | 1 | 1 | 1 | 1 | 1 | 1 |
| Abutilon | 1 | 1 | 1 | 1 | 1 | 1 | 1 | 1 | 1 | 1 | 1 | 1 |
| Sidaspinosa | 1 | 1 | 1 | 1 | 1 | 1 | 1 | 1 | 1 | 1 | 1 | 1 |
| Xanthium Sp. | - | - | - | - | 6 | 7 | 7 | 9 | 2 | 8 | 9 | 9 |
| Amaranthus ret. | 1 | 1 | 1 | 1 | 1 | 1 | 1 | 1 | 1 | 1 | 1 | 1 |
| Chenopodium Sp. | - | - | - | - | 1 | 1 | 1 | 1 | 1 | 1 | 1 | 1 |
| Solanum nigrum | 1 | 1 | 1 | 1 | 1 | 1 | 1 | 1 | 1 | 1 | 1 | 1 |
| Ipomosa | 1 | 2 | 8 | 8 | 1 | 1 | 2 | 7 | 1 | 1 | 1 | 3 |
| Sinapis | 1 | 1 | 1 | 1 | 1 | 1 | 1 | 1 | 1 | 1 | 1 | 1 |
| Stellaria | 1 | 1 | 1 | 1 | 1 | 1 | 1 | 1 | 1 | 1 | 1 | 1 |
| Chrysanthe. leuc. | 2 | 3 | 7 | 7 | 1 | 2 | 6 | 9 | 1 | 3 | 7 | 9 |
| Galium aparine | 1 | 2 | 3 | 4 | 1 | 1 | 2 | 4 | 1 | 1 | 1 | 4 |
| Viola tricolor | - | - | - | - | 1 | 1 | 1 | 1 | 1 | 1 | 1 | 1 |

Pre-emergente Herbizid-Wirkung (Fortsetzung)

| Wirkung | | | | | | | | | | | | |
|---|---|---|---|---|---|---|---|---|---|---|---|---|
| Aufwandmenge kg AS/ha | Verb. Nr. 35 | | | | Verb. Nr. 50 | | | | Verb. Nr. 51 | | | |
| Testpflanze | 4 | 2 | 1 | 0,5 | 4 | 2 | 1 | 0,5 | 4 | 2 | 1 | 0,5 |
| Veronica Sp. | - | - | - | - | 1 | 1 | 1 | 1 | 1 | 1 | 1 | 1 |
| Portulaca Sp. | 1 | 1 | 1 | 1 | - | - | - | - | - | - | - | - |
| Sesbania ex. | 1 | 1 | 8 | 9 | - | - | - | - | - | - | - | - |
| Kochia scop. | 1 | 1 | 1 | 1 | - | - | - | - | - | - | - | - |

Beispiel 3

Nachweis der Herbizidwirkung nach dem Auflaufen der Pflanzen (Kontaktwirkung).

Eine Anzahl Unkräuter und Kulturpflanzen, sowohl monocotyle wie dicotyle, wurden nach dem Auflaufen, im 4- bis 6-Blattstadium mit einer wässrigen Wirkstoffdispersion in Dosierungen von 4 kg/AS/ha gespritzt und dann bei 24° bis 26 °C und 45-60 % relativer Luftfeuchtigkeit gehalten. 15 Tage nach der Behandlung wird der Versuch ausgewertet und die Wirkung nach dem gleichen Massstab wie im Beispiel 1 bewertet.

Aus in diesem Versuch zeigten die Verbindungen der Formel I ausgezeichnete Wirkung gegen zweikeimblättrige und die meisten der grasartigen Unkräuter, wobei die Kulturpflanze wie Getreide, Mais, Hirse, Reis, Baumwolle und Soja nicht oder erst mit höheren Aufwandmengen an Wirkstoff geschädigt wurden.

Post-emergente Wirkung

Aufwandmenge : 4 kg Wirksubstanz/Hektar

| Verb. Nr. | Setaria | Solanum | Sinapis | Stellaria |
|---|---|---|---|---|
| 8 | 3 | 1 | 2 | 2 |
| 29 | 2 | 2 | 2 | 2 |
| 30 | 4 | 2 | 2 | 3 |
| 35 | 2 | 1 | 2 | 2 |
| 50 | 2 | 1 | 2 | 2 |
| 51 | 2 | 1 | 1 | 2 |

Beispiel 4

Vergleichstest zum Nachweis verbesserter Kulturpflanzenverträglichkeit.

In der gleichen Versuchsanordnung wie im Beispiel 1 werden Samen von verschiedenen Getreidesorten und den Hauptunkräutern von Getreidekulturen mit verschiedenen Aufwandmengen an Wirksubstanz behandelt. Die Auswertung erfolgte nach 3 Wochen.

Als Vergleichswirkstoffe werden die

Verbindung Nr. 35 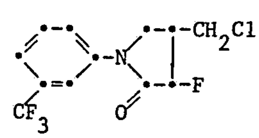

und die aus DOS 2 612 731 bekannte

Verbindung A 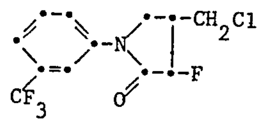

verwendet.

Die in der folgenden Tabelle aufgeführten Resultate sind in Prozent Wachstum im Vergleich zur unbehandelten Kontrolle angegeben.

Pre-emergenter Vergleichsversuch in Getreidekulturen

| Wachstum in % | Verb. Nr. 35 | | | Verb. A | | |
|---|---|---|---|---|---|---|
| Testpflanze | 2 | 1 | 0,5 kg AS/ha | 2 | 1 | 0,5 kg AS/ha |
| Sommerweizen "Sveno" | 75 | 90 | 100 | 40 | 50 | 90 |
| Winterweizen "Probus" | 75 | 100 | 100 | 60 | 75 | 90 |
| Wintergerste "Nymphe" | 90 | 90 | 100 | 25 | 50 | 90 |
| Alopecurus myos. | 0 | 0 | 50 | 0 | 0 | 0 |
| Sinapis alba | 0 | 0 | 0 | 0 | 0 | 0 |
| Matricaria cham. | 0 | 0 | 0 | 0 | 0 | 0 |
| Stellaria media | 0 | 0 | 0 | 0 | 0 | 0 |
| Veronica pers. | 0 | 0 | 0 | 0 | 0 | 0 |
| Viola tricolor | 0 | 0 | 0 | 0 | 0 | 10 |

Aus den Testergebnissen ist ersichtlich, dass die Verbindung Nr. 35 von den Getreidepflanzen bei höheren Aufwandmengen besser toleriert wird, als die Vergleichsverbindung A.

**Ansprüche**

1. Fluorpyrrolidinone der allgemeinen Formel I

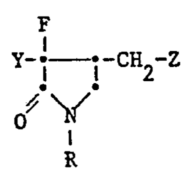

(I)

worin

R $C_1$-$C_4$-Alkyl ; $C_3$-$C_4$-Alkenyl ; Alkoxyalkyl mit insgesamt höchstens 5 Kohlenstoffatomen ; gegebenenfalls durch Halogen, $C_1$-$C_4$-Alkoxy oder $C_1$-$C_4$-Alkyl substituiertes Benzyl ; oder ein- oder mehrfach durch Halogen, Nitro, Cyan, Methyl, Halogenmethyl, $C_1$-$C_4$-Alkoxy, $C_1$-$C_4$-Halogenalkoxy, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl substituiertes Phenyl bedeutet und

Y für Wasserstoff, $C_1$-$C_4$-Alkyl, Fluor, Chlor oder Brom und

Z für Chlor oder Brom stehen.

2. Verbindungen der Formel I, gemäss Anspruch 1, dadurch gekennzeichnet, dass R einen ein- oder mehrfach durch Halogen, Nitro, Cyan, Methoxy, Halogenmethoxy, Methyl, Halogenmethyl, Halogenmethylthio, Halogenmethylsulfinyl oder Halogenmethylsulfonyl substituierten Phenylkern, Y Wasserstoff, Methyl, Chlor oder Brom und Z Chlor oder Brom bedeuten.

3. Verbindungen der Formel I, gemäss Anspruch 2, dadurch gekennzeichnet, dass R einen ein- oder mehrfach durch Halogen, Halogenmethyl oder Halogenmethoxy substituierten Phenylkern, Y Wasserstoff oder Chlor bedeuten und Z für Chlor steht.

4. Verbindungen gemäss Anspruch 3, dadurch gekennzeichnet, dass R einen in 3-Stellung durch

Halogen, Trifluormethyl, Trifluormethoxy oder Difluormethoxy substituierten Phenylkern, Y Wasserstoff oder Chlor und Z Chlor bedeuten.

5. Die Verbindung N-(3-Trifluormethylphenyl)-3-fluor-4-chlormethylpyrrolidin-2-on unter Einschluss ihrer diastereomeren Formen.

6. Verfahren zur Herstellung der Verbindungen der Formel I, gemäss Anspruch 1, dadurch gekennzeichnet, dass man ein N-Allyl-acetamid der Formel II,

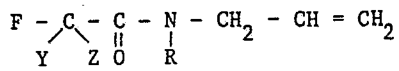

(II)

worin R, Y und Z die unter Formel I angegebene Bedeutung haben, in Gegenwart eines Katalysators in einem inerten Lösungsmittel erhitzt.

7. Herbizides Mittel, dadurch gekennzeichnet, dass es neben Träger- und/oder anderen Zuschlagstoffen als Wirkstoff mindestens ein Fluorpyrrolidinon der Formel I, gemäss Anspruch 1, enthält.

8. Herbizides Mittel gemäss Anspruch 7, dadurch gekennzeichnet, dass es als Wirkstoff Verbindungen gemäss einem der Ansprüche 2 bis 5 enthält.

9. Verwendung der Verbindungen der Formel I, gemäss Anspruch 1, oder sie enthaltender Mittel als selektive Herbizide.

10. Verwendung der Fluorpyrrolidinone gemäss Anspruch 9 zur pre- oder post-emergenten Bekämpfung von Unkräutern in Nutzpflanzenkulturen.

11. Verwendung gemäss Anspruch 10 zur Bekämpfung von Unkräutern in Getreide-, Reis- und Baumwollkulturen.

## Claims

1. A fluoropyrrolidinone of the general formula I

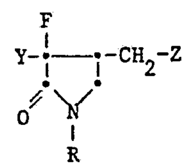

(I)

wherein R is $C_1$-$C_4$-alkyl, $C_3$-$C_4$-alkenyl, alkoxyalkyl of at most 5 carbon atoms, benzyl or benzyl substituted by halogen, $C_1$-$C_4$-alkoxy or $C_1$-$C_4$-alkyl, or is phenyl or phenyl monosubstituted or polysubstituted by halogen, nitro, cyano, methyl, halomethyl, $C_1$-$C_4$-alkoxy, $C_1$-$C_4$-haloalkoxy, $C_1$-$C_4$-haloalkylthio, $C_1$-$C_4$-alkylsulfinyl, $C_1$-$C_4$-haloalkylsulfinyl, $C_1$-$C_4$-alkylsulfonyl or $C_1$-$C_4$-haloalkylsulfonyl ; Y is hydrogen, $C_1$-$C_4$-alkyl, fluorine, chlorine or bromine ; and Z is chlorine or bromine.

2. A compound of the formula I, according to claim 1, wherein R is a phenyl nucleus which is monosubstituted or polysubstituted by halogen, nitro, cyano, methoxy, halomethoxy, methyl, halomethyl, halomethylthio, halomethylsulfinyl or halomethylsulfonyl, Y is hydrogen, methyl, chlorine or bromine, and Z is chlorine or bromine.

3. A compound of the formula I according to claim 2, wherein R is a phenyl nucleus which is monosubstituted or polysubstituted by halogen, halomethyl or halomethoxy, Y is hydrogen or chlorine, and Z is chlorine.

4. A compound according to claim 3, wherein R is a phenyl nucleus which is substituted in the 3-position by halogen, trifluoromethyl, trifluoromethoxy or difluoromethoxy, Y is hydrogen or chlorine, and Z is chlorine.

5. N-(3-Trifluoromethylphenyl)-3-fluoro-4-chloromethylpyrrolidin-2-one and the diastereoisomers thereof.

6. A process for the preparation of a compound of the formula I according to claim 1, which process comprises heating an N-allylacetamide of the formula II

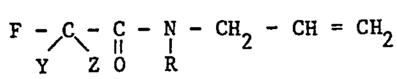

(II)

wherein R, Y and Z are as defined for formula I, in the presence of a catalyst and in an inert solvent.

7. A herbicidal composition containing at least one fluoropyrrolidinone of the formula I according to claim 1, together with solid carriers and/or other adjuvants.

8. A herbicidal composition according to claim 7, which contains as active ingredient a compound according to any one of claims 2 to 5.

9. Use of a compound of the formula I according to claim 1, or of a composition containing such a compound, as selective herbicide.

10. Use of a fluoropyrrolidinone according to claim 9 for controlling weeds pre- or postemergence in crops of useful plants.

11. Use according to claim 10 for controlling weeds in crops of cereals, rice and cotton.

**Revendications**

1. Fluoropyrrolidinones de formule générale I

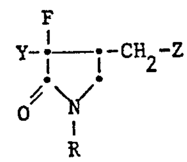

(I)

où

R représente un alcoyle en $C_1$ à $C_4$ ; un alcényle en $C_3$ à $C_4$ ; un alcoxyalcoyle avec au total au plus 5 atomes de carbone ; un benzyle éventuellement substitué par un halogène, un alcoxy en $C_1$ à $C_4$ ou un alcoyle en $C_1$ à $C_4$ ; ou un phényle mono- ou polysubstitué par un halogène, un nitro, un cyano, un méthyle, un halogénométhyle, un alcoxy en $C_1$ à $C_4$, un halogénalcoxy en $C_1$ à $C_4$, un halogénalcoylthio en $C_1$ à $C_4$, un alcoyle en $C_1$ à $C_4$-sufinyle, un halogène-alcoyle en $C_1$ à $C_4$-sulfinyle, un alcoyle en $C_1$ à $C_4$-sulfonyle ou un halogène-alcoyle en $C_1$ à $C_4$-sulfonyle et

Y représente un hydrogène, un alcoyle en $C_1$ à $C_4$, un fluor, un chlore ou un brome et

Z représente un chlore ou un brome.

2. Composés de formule I selon la revendication 1, caractérisés en ce que R représente un noyau phényle mono- ou polysubstitué par un halogène, un nitro, un cyano, un métoxy, un halogène-méthoxy, un méthyle, un halogèneméthyle, un halogèneméthylthio, un halogèneméthylsulfinyle ou un halogène-méthylsulfonyle, Y représente un hydrogène, un méthyle, un chlore ou un brome et Z représente un chlore ou un brome.

3. Composés de formule I selon la revendication 2, caractérisés en ce que R représente un noyau phényle mono- ou polysubstitué par un halogène, un halogénométhyle ou un halogèneméthoxy, Y représente un hydrogène ou un chlore et Z représente un chlore.

4. Composés selon la revendication 3, caractérisés en ce que R représente un noyau phényle substitué en position 3 par un halogène, un trifluorométhyle, un trifluorométhoxy ou un difluorométhoxy, Y représente un hydrogène ou un chlore et Z représente un chlore.

5. Le composé N-(3-trifluorométhylphényl)-3-fluoro-4-chlorométhyl-pyrrolidin-2-one y compris ses formes diastéréomères.

6. Procédé de préparation des composés de formule I selon la revendication 1, caractérisé en ce qu'on chauffe un N-allyl-acétamide de formule II

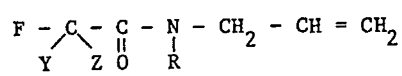

(II)

où R, Y et Z ont la signification donnée pour la formule I, en présence d'un catalyseur dans un solvant inerte.

7. Agent herbicide, caractérisé en ce qu'il contient, outre des supports et/ou autres additifs, comme matière active au moins une fluoropyrrolidinone de formule I selon la revendication 1.

8. Agent herbicide selon la revendication 7, caractérisé en ce qu'il contient comme matière active des composés selon l'une des revendications 2 à 5.

9. Application des composés de formule I selon la revendication 1 ou des agents qui les contiennent comme herbicides sélectifs.

10. Application des fluoropyrrolidinones selon la revendication 9 à la lutte en pré- ou en post-levée contre les mauvaises herbes dans les cultures de plantes utiles.

11. Application selon la revendication 10 à la lutte contre les mauvaises herbes dans les cultures de céréales, de riz et de coton.